(19) **Europäisches Patentamt**
**European Patent Office**
**Office européen des brevets**

(11) **EP 1 853 338 B1**

(12) **EUROPEAN PATENT SPECIFICATION**

(45) Date of publication and mention
of the grant of the patent:
**17.07.2019  Bulletin 2019/29**

(51) Int Cl.:
*A61M 15/08* *(2006.01)*   *A61M 15/00* *(2006.01)*
*A61K 9/48* *(2006.01)*   *A61J 3/07* *(2006.01)*

(21) Application number: **06709863.2**

(22) Date of filing: **23.02.2006**

(86) International application number:
**PCT/GB2006/000631**

(87) International publication number:
**WO 2006/090149 (31.08.2006 Gazette 2006/35)**

(54) **NASAL DELIVERY DEVICE**

NASALES VERABREICHUNGSGERÄT

DISPOSITIFS D'ADMINISTRATION DE POUDRE

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(30) Priority:  **23.02.2005   GB 0503738**

(43) Date of publication of application:
**14.11.2007   Bulletin 2007/46**

(73) Proprietor: **Optinose AS**
**0349 Oslo (NO)**

(72) Inventors:
  • **DJUPESLAND, Per Gisle**
  **N-0349 Oslo (NO)**
  • **SHELDRAKE, Colin David**
  **South Marston Park, Wiltshire SN3 4TG (GB)**
  • **HAFNER, Roderick Peter**
  **South Marston Park, Wiltshire SN3 4TG (GB)**

(74) Representative: **Boden, Keith McMurray**
**Fry Heath & Spence LLP**
**Unit A, Faraday Court**
**Faraday Road**
**Crawley, West Sussex RH10 9PU (GB)**

(56) References cited:
**WO-A-00/51672**   **WO-A-03/090812**
**WO-A-2004/060433**   **WO-A-2004/082750**
**WO-A-2004/091705**   **WO-A-2004/103447**
**WO-A-2005/021059**   **WO-A-2006/030210**
**DE-A1- 10 300 982**   **DE-A1- 19 835 346**
**GB-A- 2 404 867**   **US-A- 5 373 841**
**US-A1- 2004 043 064**

**Description**

The present invention relates to a powder delivery device for the delivery of a powdered substance, in particular to the nasal airway, and both a powdered substance and a capsule for use with the same.

There is an increasing interest in the nasal delivery of substances, typically pharmaceutical drugs, both as powders and liquids, for topical and systemic delivery.

Current delivery systems are not suited to the delivery of substances to the upper posterior region of the nasal airway, in particular targeted delivery to the olfactory region and the sinus ostia.

US-A-4013075 and US-A-4889114 disclose examples of prior art inhalation devices, which provide for the inhalation of a powdered substance from a capsule.

WO-A-00/051672 discloses a delivery device for delivering a substance, in particular a medicament, in a bi-directional flow through the nasal cavities, that is, an air flow which passes into one nostril, around the posterior margin of the nasal septum and in the opposite direction out of the other nostril. A particular feature of this bi-directional mode of delivery is the ability to target defined regions in the nasal airway, for both topical and systemic delivery, in particular the upper posterior region which cannot be targeted with existing systems.

WO-A-2003/090812 discloses a nasal delivery device for delivering substance to a nasal airway of a subject.

WO-A-2004/103447 discloses a delivery device for delivering substance to a mucosal surface within the oral cavity of a subject.

GB-A-2404867 discloses a breath-actuated nasal delivery device for delivering substance to a nasal airway of a subject.

WO-A-2004/060433 discloses a delivery device for delivering substance to a subject through nasal mucosa in a nasal cavity of a subject.

US-A-5373841 discloses a self-operated nasal humidifier comprising a container adapted to hold heated liquid and a lid detachably secured to the container.

The present inventors have recognized that the delivery of powdered substances using the exhalation breath of a subject still presents a significant challenge, owing to the interaction of the moist exhaled air flow with the powdered substance prior to delivery into the nasal airway.

Exhalation into a device leads to condensation on the surfaces of the exposed device components, where the components are at a significantly lower temperature than the exhaled air flow, and significant condensation in the delivery channel will affect the consistency of the delivered doses.

It is an aim of the present invention to provide a delivery device which allows for the delivery of powdered substances, either supplied in capsules or blisters, which contain a pre-metered dose of substance with the appropriate particle size distribution and surface properties, or metered from bulk, where using the exhalation breath of the subject.

In one aspect the present invention provides a nasal delivery device for delivering substance to a nasal cavity of a subject according to claim 1.

In one embodiment the mouthpiece unit includes a plurality of temperature modifiers which can be fluidly connected successively to the mouthpiece, and a switching mechanism which allows for one of the temperature modifiers to be fluidly connected to the mouthpiece.

Preferably, when the one of the temperature modifiers is fluidly connected to the mouthpiece, the at least one other temperature modifier is vented to atmosphere.

In one embodiment the switching mechanism comprises a rotatable member to which the temperature modifiers are disposed, whereby rotation of the switching mechanism provides for the one of the temperature modifiers to be in fluid communication with the mouthpiece.

In one embodiment the container chamber is substantially cylindrical in shape.

In another embodiment the container chamber is substantially spherical in shape.

In one embodiment the substance supply unit comprises a rupturing mechanism for rupturing the container as contained in the container chamber.

In one embodiment the container is formed of a material which exhibits insufficient tackiness, and preferably substantially no surface tackiness, in the presence of moisture such as not to adhere to an inner surface of the container chamber during emptying of the container.

Preferably, the container is formed of a material which exhibits insufficient tackiness in the presence of moisture in the exhalation air flow for a period of up to about 5 s following exhalation.

More preferably, the container is formed of a material which exhibits insufficient tackiness in the presence of moisture in the exhalation air flow for a period of up to about 2 s following exhalation.

Still more preferably, the container is formed of a material which exhibits insufficient tackiness in the presence of moisture in the exhalation air flow for a period of up to about 1 s following exhalation.

In one embodiment the container is formed substantially of a cellulose derivative.

Preferably, the container is formed substantially of one of hydroxypropyl methylcellulose (HPMC), hydroxypropylcellulose, methylcellulose, ethylcellulose and carboxymethylcellulose.

In another embodiment the container is formed substantially of gelatine.

In a further embodiment the container is formed of a plastics material.

In a still further embodiment the container includes a coating of a material which exhibits insufficient tackiness in the presence of moisture such as not to adhere to an inner surface of the container chamber during emptying of the container.

Preferably, the coating is formed of a material which exhibits insufficient tackiness in the presence of moisture in the exhalation air flow for a period of up to about 5 s following exhalation.

More preferably, the coating is formed of a material which exhibits insufficient tackiness in the presence of moisture in the exhalation air flow for a period of up to about 2 s following exhalation.

Still more preferably, the coating is formed of a material which exhibits insufficient tackiness in the presence of moisture in the exhalation air flow for a period of up to about 1 s following exhalation.

Preferably, the coating comprises substantially one of parylene, hydroxypropyl methylcellulose (HPMC), hydroxypropylcellulose, methylcellulose, ethylcellulose, carboxymethylcellulose, polyvinyl alcohol, acrylic acid polymer, methacrylic acid polymer, ethyl acrylic acid polymer, cellulose acetate phthalate, polyvinyl acetate phthalate, hydroxypropyl methylcellulose phthalate and hydroxyl methylcellulose acetate succinate, or any combination of layers thereof.

In one embodiment the container comprises a body of gelatine.

In one embodiment the container comprises a capsule.

In one embodiment the capsule is substantially cylindrical in shape.

In another embodiment the capsule is substantially spherical in shape.

Preferably, the at least one temperature modifier is configured to allow a flow therethrough at a flow rate of at least about 10 l/min at a pressure of less than about 1 kPa.

More preferably, the at least one temperature modifier is configured to allow a flow therethrough at a flow rate of at least about 20 l/min at a pressure of less than about 2 kPa, and preferably less than about 1 kPa.

Still more preferably, the at least one temperature modifier is configured to allow a flow therethrough at a flow rate of at least about 30 l/min at a pressure of less than about 2 kPa, and preferably less than about 1 kPa.

Yet more preferably, the at least one temperature modifier is configured to allow a flow therethrough at a flow rate of at least about 40 l/min at a pressure of less than about 2 kPa, and preferably less than about 1 kPa.

Still yet more preferably, the at least one temperature modifier is configured to allow a flow therethrough at a flow rate of at least about 50 l/min at a pressure of less than about 2 kPa, and preferably less than about 1 kPa.

Preferably, the at least one temperature modifier is configured such as to provide a pressure drop of not more than about 0.25 kPa to the exhaled air flow.

Still more preferably, the at least one temperature modifier is configured such as to provide a pressure drop of not more than about 0.10 kPa to the exhaled air flow.

Yet more preferably, the at least one temperature modifier is configured such as to provide a pressure drop of not more than about 0.05 kPa to the exhaled air flow.

Still yet more preferably, the at least one temperature modifier is configured such as to provide a pressure drop of not more than about 0.025 kPa to the exhaled air flow.

In another embodiment the at least one temperature modifier comprises a thermoelectric device.

Preferred embodiments of the present invention will now be described hereinbelow by way of example only with reference to the accompanying drawings. The delivery devices of Figures 6 to 15 represent devices which do not constitute part of the present invention.

Figure 1 illustrates a delivery device in accordance with a first embodiment of the present invention;

Figure 2 illustrates the heat exchanger of the delivery device of Figure 1;

Figure 3 illustrates the delivery device of Figure 1, in the operative state;

Figure 4 illustrates the mouthpiece unit of a delivery device as a modification of the delivery device of Figure 1, in a first operative configuration;

Figure 5 illustrates the mouthpiece unit of Figure 4, in a second operative configuration;

Figure 6 illustrates an example of a delivery device;

Figure 7 illustrates the delivery device of Figure 6, in the operative state;

Figure 8 illustrates a delivery device as a modification of the delivery device of Figure 6;

Figure 9 illustrates the delivery device of Figure 8, in the operative state;

Figure 10 illustrates another example of a delivery device;

Figure 11 illustrates the delivery device of Figure 10, in the operative state;

Figure 12 illustrates a still further example of a delivery device;

Figure 13 illustrates the delivery device of Figure 12, in a first operative state;

Figure 14 illustrates the delivery device of Figure 12, in a second operative state; and

Figure 15 illustrates a delivery device as one modification of the delivery device of Figure 12.

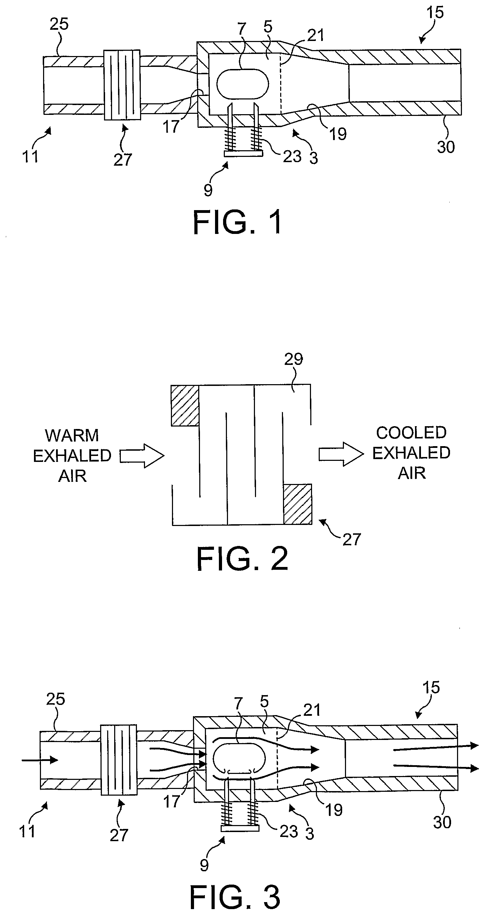

Figures 1 to 3 illustrate a delivery device in accordance with a first embodiment of the present invention.

The delivery device comprises a substance supply unit 3 which includes a chamber 5 which receives a capsule 7, which contains a metered amount of a powdered substance which is to be delivered by the delivery device, a rupturing mechanism 9 for rupturing the capsule 7, a mouthpiece unit 11 which is in fluid communication with the chamber 5 and is gripped in use in the mouth of a subject, and a nosepiece unit 15 which is in fluid communication with the chamber 5 and is fitted to one nostril of the subject. For ease of illustration, the delivery device is illustrated in an elongate configuration, but, in its practical embodiment, the mouthpiece unit 11 and the nosepiece unit 15 are configured for fitting to the mouth and one nostril of the subject.

The substance supply unit 3 includes an inlet 17 which fluidly connects the chamber 5 thereof with the mouthpiece unit 11 and an outlet 19 which fluidly connects the chamber 5 thereof with the nosepiece unit 15.

In this embodiment the substance supply unit 3 includes a grid 21, here a gauze, which is disposed at the outlet 19 thereof and acts to prevent the capsule 7 or parts thereof from escaping from the chamber 5.

In this embodiment the chamber 5 is cylindrical in shape.

In another embodiment the chamber 5 can be substantially spherical in shape, which is particularly advantageous in allowing for the release of the powdered substance from the capsule 7 in any operative position.

In this embodiment the chamber 5 and the grid 21, as components which contact the capsule 7 and the contained powder, are fabricated from a material having a low moisture sensitivity, here a plastics material, such as to reduce any tendency to become tacky in the presence of moisture, and therefore reduce the tendency for the capsule 7 and the powdered substance as contained thereby to adhere to the wall of the chamber 5 or the grid 21.

In this embodiment the rupturing mechanism 9 comprises a piercing element 23, here including two pins, which is operable to pierce the capsule 7, and thereby provide for the release of the contained powdered substance on the generation of a flow through the chamber 5.

The mouthpiece unit 11 comprises a mouthpiece 25, in this embodiment as defined by a tubular section, which is gripped in the mouth of the subject, and a heat exchanger 27 which is in fluid communication with the mouthpiece 25 and acts to draw heat from the exhaled air flow as delivered through the mouthpiece 25, thus decreasing the temperature of the air flow as delivered to the chamber 5. By decreasing the temperature of the air flow, the humidity of the air flow is reduced, with the water vapor condensing in the heat exchanger 27, and the impact of condensation is significantly reduced, thus allowing for successive doses of powdered substance to be delivered without affecting the release of powdered substance from the capsules 7.

As illustrated in Figure 2, in this embodiment the heat exchanger 27 comprises a channel 29 which has a zig-zag, serpentine configuration, with a circular cross section. In other embodiments the channel 29 could have other configurations, for example, a rectangular cross section.

In this embodiment the channel 29 has an effective length of 200 mm and an effective diameter of 4 mm, which reduces the temperature of an exhaled air flow which has a flow rate of 30 l/min to about 25 °C from about 37 °C, where the channel 29 is at a temperature of 20 °C.

The reduction in temperature is calculated as follows:

$$T_e = T_w - (T_w - T_I)e^{-hAmC}$$

Where:

$T_e$ is the fluid temperature at the exit of the channel 29;

$T_w$ is the fluid temperature at the wall of the channel 29;

$T_i$ is the fluid temperature at the inlet of the channel 29;

h is the heat transfer coefficient between the gas flowing through the channel 29 and the material of the channel 29;

A is the surface area of the channel 29;

m is the mass flow rate; and

C is the specific heat capacity of the gas flowing through the channel 29.

This calculation assumes turbulent flow in the channel 29 ($Nu = 0.023Re^{0.8}Pr^{0.3}$).

In other embodiments the channel 29 can include features to enhance the heat transfer coefficient from the exhaled air flow to the wall of the channel 29, such that the effective length of the channel 29 can be considerably reduced. Typical features include nodules or areas of relative surface roughness that create turbulence and so enhance the heat transfer.

In other embodiments the heat exchanger 27 could comprise a plurality of channels 29.

In one embodiment the heat exchanger 27 comprises four channels 29, as parallel ducts, which each have a width of 10 mm, a height of 1.5 mm and a length of 60 mm. This configuration reduces the temperature of an exhaled air flow which has a flow rate of 30 l/min by about 5 °C, where the channels 29 are at a temperature of 20 °C, and also cause only a very small pressure drop of 0.024 kPa.

The nosepiece unit 15 comprises a nosepiece 30, in this embodiment as defined by a tubular section, which is inserted into a nostril of the subject, in this embodiment to provide a sealing fit therewith.

In this embodiment the nosepiece 30, as a component which contacts the powdered substance, is fabricated from a material having a low moisture sensitivity, here a plastics material, such as to reduce any tendency to become tacky in the presence of moisture, and therefore reduce the tendency for the powdered substance to adhere to the wall of the nosepiece 30.

In one embodiment the capsule 7 is a gelatine capsule.

In another embodiment the capsule 7 can be manufactured from a material which has a reduced tendency to become tacky in the presence of moisture, as occurs with gelatine capsules, and therefore reduce the tendency for the capsule 7 to adhere to the wall of the chamber 5 or the grid 21.

In one embodiment the capsule 7 is formed of a cellulose derivative, such as hydroxypropyl methylcellulose (HPMC), hydroxypropylcellulose, methylcellulose, ethylcellulose and carboxymethylcellulose

In another embodiment the capsule 7 can comprise a plastics material, preferably a water insoluble material, such as a polycarbonate.

In one embodiment the capsule 7 can be manufactured from a lightweight material, such as thin-wall section polymeric materials, which reduces the energy required to move the capsule 7, typically by one or both of vibration and rotation, and thereby allow the delivery device to be operated at reduced flow rates, which is particularly advantageous for nasal delivery.

In one embodiment the capsule 7 has a wall section of less than about 0.25 mm, and more preferably less than about 0.2 mm.

In an alternative embodiment the capsule 7 can include an outer coating of a material which has a reduced tendency to become tacky in the presence of moisture, as occurs with gelatine capsules, and therefore reduce the tendency for the capsule 7 to adhere to the wall of the chamber 5 or the grid 21.

In one embodiment the coated capsule 7 can be formed of gelatine.

In one embodiment the coating can comprise one of parylene, hydroxypropyl methylcellulose (HPMC), hydroxypropylcellulose, methylcellulose, ethylcellulose, carboxymethylcellulose, polyvinyl alcohol, acrylic acid polymer, methacrylic acid polymer, ethyl acrylic acid polymer, cellulose acetate phthalate, polyvinyl acetate phthalate, hydroxypropyl methylcellulose phthalate and hydroxyl methylcellulose acetate succinate.

The delivery device of this embodiment is operative to discharge the powdered substance from the capsule 7 by rotation and vibration of the capsule 7, and thus the capsule 7 is preferably formed of a material or coated with a material which exhibits substantially no tackiness in the presence of a moist environment, here a saturated exhaled air flow, that is, does not exhibit an increased moisture content at the outer surface thereof, which would prevent reliable rotation and vibration of the capsule 7.

In this embodiment, as illustrated in Figure 1, the capsule 7 is cylindrical in shape with hemispherical ends.

In other embodiments the capsule 7 could have other geometric forms, such as spherical, which allows for efficient powder release at low flow rates.

In one embodiment the capsule 7 can comprise two or more parts.

In one alternative embodiment the capsule 7 can be constructed to act as the primary environmental barrier for the powdered substance. For example, the capsule 7 could be constructed from a relatively thick-walled cylindrical section of a polymeric material which includes two metalized thin film closure members which act to seal the ends of the cylindrical section and thus enclose the same.

In one embodiment, where the delivery device is a re-usable device, the chamber 5, which contains the capsule 7, and the nosepiece 30 comprise a unitary, replaceable component.

In operation, as illustrated in Figure 3, a subject operates the rupturing mechanism 9 to rupture the capsule 7, inserts the nosepiece 30 into one of his/her nostrils, grips the mouthpiece 25 in his/her mouth, and exhales through the mouthpiece 25.

The exhaled air flow is reduced in temperature by the heat exchanger 27 on delivery therethrough, such as to reduce the absolute humidity of the exhaled air flow, and this cooled air is then driven through the chamber 5, which acts to move the capsule 7, in this embodiment by vibration and rotation, and entrain the powdered substance as contained by the capsule 7.

The exhaled air flow, as then entraining the powdered substance, is delivered though the nosepiece 30 into one nasal cavity of the subject.

In this embodiment the exhaled air flow has such a pressure as to pass around the posterior region of the nasal septum, and into the other nasal cavity, thereby achieving a bi-directional air flow as described in the applicants' earlier WO-A-00/051672.

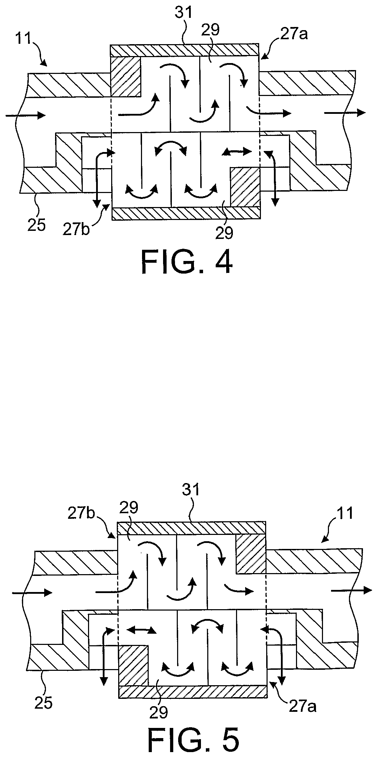

In one modification, as illustrated in Figures 4 and 5, the mouthpiece unit 11 includes a plurality of, in this embodiment first and second heat exchangers 27a, b which can be used successively, such as to allow for the evaporation of the condensed moisture from the one or more previously-used heat exchangers 27a, b, and a switching mechanism 31 which allows for one of the heat exchangers 27a, b to be fluidly connected to the mouthpiece 25.

In this embodiment the switching mechanism 31 comprises a rotatable member to which the heat exchangers 27a, b are disposed, whereby rotation of the switching mechanism 31 provides for one of the heat exchangers 27a, b to be in fluid communication with the mouthpiece 25 and the at least one other of the heat exchangers 27a, b to be in fluid communication with the atmosphere. Figure 4 illustrates a first configuration, in which the first heat exchanger 27a is in fluid communication with the mouthpiece 25 and the second heat exchanger 27b is vented to atmosphere. Figure 5 illustrates a second configuration, in which the second heat exchanger 27b is in fluid communication with the mouthpiece 25 and the first heat exchanger 27a is vented to atmosphere.

With this configuration, the one of the heat exchangers 27a, b which is in fluid communication with the mouthpiece 25 acts to cool the exhaled air flow as delivered therethrough, and thereby trap water vapor from the exhaled air, and the other of the heat exchangers 27a, b which is vented to atmosphere provides for evaporation of the water condensate as trapped from a previous exhalation therethrough.

In an alternative embodiment the switching mechanism 31 could be operatively coupled to the rupturing mechanism 9, such as to provide for operation of the switching mechanism 31 with each operation of the rupturing mechanism 9.

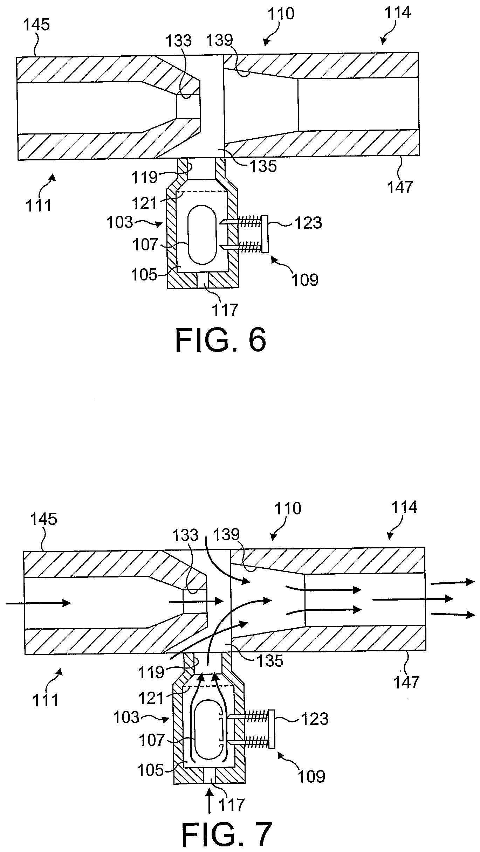

Figures 6 and 7 illustrate another nasal delivery device.

The delivery device comprises a substance supply unit 103 which includes a chamber 105 which receives a capsule 107, which contains a metered amount of a powdered substance which is to be delivered by the delivery device, a rupturing mechanism 109 for rupturing the capsule 107, a Venturi unit 110 which is in fluid communication with the chamber 105 and is operative to draw an air flow of the ambient atmosphere through the chamber 105, a mouthpiece unit 111 which is in fluid communication with the Venturi unit 110 and is gripped in use in the mouth of a subject, and a nosepiece unit 114 which is in fluid communication with the Venturi unit 110 and is fitted to one nostril of the subject. For ease of illustration, the delivery device is illustrated in an elongate configuration, but, in its practical design, the mouthpiece unit 111 and the nosepiece unit 114 are configured for fitting to the mouth and one nostril of the subject.

The substance supply unit 103 includes an inlet 117 which fluidly connects the chamber 105 thereof with the ambient atmosphere and an outlet 119 which fluidly connects the chamber 105 thereof with the Venturi unit 110.

In this design the substance supply unit 103 includes a grid 121, here a gauze, which is disposed at the outlet 119 thereof and acts to prevent the capsule 107 or parts thereof from escaping from the chamber 105.

In this design the chamber 105 is cylindrical in shape.

In another design the chamber 105 could be spherical in shape, which is particularly advantageous in allowing for the release of the powdered substance from the capsule 107 when in any operative position.

In this design the chamber 105 and the grid 121, as components which contact the capsule 107 and the contained powdered substance, are fabricated from a material having a low moisture sensitivity, here a plastics material, such as to reduce any tendency to become tacky in the presence of moisture, and therefore reduce the tendency for the capsule 107 and the powdered substance as contained thereby to adhere to the wall of the chamber 105 or the grid 121.

In this design the rupturing mechanism 109 comprises a piercing element 123, here including two pins, which is operable to pierce the capsule 107, and thereby provide for the release of the contained powdered substance on the generation of a flow through the chamber 105.

In one design the capsule 107 is a gelatine capsule.

In another design the capsule 107 can be manufactured from a material which has a reduced tendency to become tacky in the presence of moisture, as occurs with gelatine capsules, and therefore reduce the tendency for the capsule 107 to adhere to the wall of the chamber 105 or the grid 121.

In one design the capsule 107 is formed of a cellulose derivative, such as hydroxypropyl methylcellulose (HPMC), hydroxypropylcellulose, methylcellulose, ethylcellulose and carboxymethylcellulose

In another design the capsule 107 can comprise a plastics material, preferably a water insoluble material, such as a polycarbonate.

In one design the capsule 107 can be manufactured from a lightweight material, such as thin-wall section polymeric materials, which reduces the energy required to move the capsule 107, typically by one or both of vibration and rotation, and thereby allows the delivery device to be operated at reduced flow rates, which is particularly advantageous for nasal delivery.

In one design the capsule 107 has a wall section of less than about 0.25 mm, and more preferably less than about 0.2 mm.

In an alternative design the capsule 107 can include an outer coating of a material which has a reduced tendency to become tacky in the presence of moisture, as occurs with gelatine capsules, and therefore reduce the tendency for the capsule 107 to adhere to the wall of the chamber 105 or the grid 121.

In one design the coated capsule 107 can be formed of gelatine.

In one design the coating can comprise one of parylene, hydroxypropyl methylcellulose (HPMC), hydroxypropylcellulose, methylcellulose, ethylcellulose, carboxymethylcellulose, polyvinyl alcohol, acrylic acid polymer, methacrylic acid polymer, ethyl acrylic acid polymer, cellulose acetate phthalate, polyvinyl acetate phthalate, hydroxypropyl methylcellulose phthalate and hydroxyl methylcellulose acetate succinate, or any combination of layers thereof.

The delivery device of this design is operative to discharge the powdered substance from the capsule 107 by rotation and vibration of the capsule 107, and thus the capsule 107 is preferably formed of a material or coated with a material which exhibits substantially no tackiness in the presence of a moist environment, here a saturated exhaled air flow, that is, does not exhibit an increased moisture content at the outer surface thereof, which would prevent reliable rotation and vibration of the capsule 107.

In this design the capsule 107 is cylindrical in shape, with hemispherical ends.

In other designs the capsule 107 could have other geometric forms, such as spherical, which allows for efficient powder release at low flow rates.

In one design the capsule 107 can comprise two or more parts.

In one alternative design the capsule 107 can be constructed to act as the primary environmental barrier for the powdered substance. For example, the capsule 107 could be constructed from a relatively thick-walled cylindrical section of a polymeric material which includes two metalized thin film closure members which act to seal the ends of the cylindrical section and thus enclose the same.

The Venturi unit 110 comprises a first, driving air flow inlet 133 which is in fluid communication with the mouthpiece unit 111 and provides a constriction which acts to accelerate the exhaled air flow to deliver a driving air flow at a higher velocity, a second, substance air flow inlet 135 which is in fluid communication with the outlet 119 of the substance supply unit 103 and through which, by the reduced local pressure as developed thereat by the Venturi effect, is drawn a substance air flow from the chamber 105 of the substance supply unit 103 which entrains the powdered substance, and an air flow outlet 139 which is in fluid communication with the nosepiece unit 114 and through which the driving air flow and the substance air flow are delivered. In this design the driving air flow is directed substantially perpendicularly to the substance air flow.

This configuration, which utilizes ambient air to entrain the powdered substance from the capsule 107, is particularly advantageous, in avoiding the use of exhaled air to entrain the powdered substance. Exhaled air has a high humidity which would lead to condensation both in the chamber 105 and the capsule 107, which can cause problems in the complete entrainment of the powdered substance, both in terms of adhesion of the capsule 107 to the wall of the chamber 105 and adhesion of the powdered substance to the wall of the capsule 107, particularly where the powdered substance is a hygroscopic powder.

The mouthpiece unit 111 comprises a mouthpiece 145, in this design as defined by a tubular section, which is gripped in the mouth of the subject.

The nosepiece unit 114 comprises a nosepiece 147, in this design as defined by a tubular section, which is inserted into a nostril of the subject, in this design to provide a sealing fit therewith.

In this design the nosepiece 147, as a component which contacts the powdered substance, is fabricated from a material having a low moisture sensitivity, here a plastics material, such as to reduce any tendency to become tacky in the presence of moisture, and therefore reduce the tendency for the powdered substance to adhere to the wall of the nosepiece 147.

In one design, where the delivery device is a reusable device, the chamber 105, which contains the capsule 107, and the nosepiece 147 comprise a unitary, replaceable component.

In operation, as illustrated in Figure 7, a subject operates the rupturing mechanism 109 to rupture the capsule 107, inserts the nosepiece 147 into one of his/her nostrils, grips the mouthpiece 145 in his/her mouth, and exhales through the mouthpiece 145.

The exhaled air flow is forced through the driving air flow inlet 133 of the Venturi unit 110, which acts to deliver the exhaled air flow as a driving air flow over the substance air flow inlet 135 of the Venturi unit 110 and draw a substance air flow, which entrains powdered substance, from the chamber 105 of the substance supply unit 103. The substance air flow acts to move the capsule 107, in this design by vibration and rotation, and entrain the powdered substance as contained by the capsule 107.

The exhaled air flow, as then entraining the powdered substance, passes through the air flow outlet 139 of the Venturi unit 110, and is delivered though the nosepiece 147 into one nasal cavity of the subject.

In this design the exhaled air flow has such a pressure as to pass around the posterior margin of the nasal septum, and into the other nasal cavity, thereby achieving a bi-directional air flow as described in the applicants' earlier WO-A-00/051672.

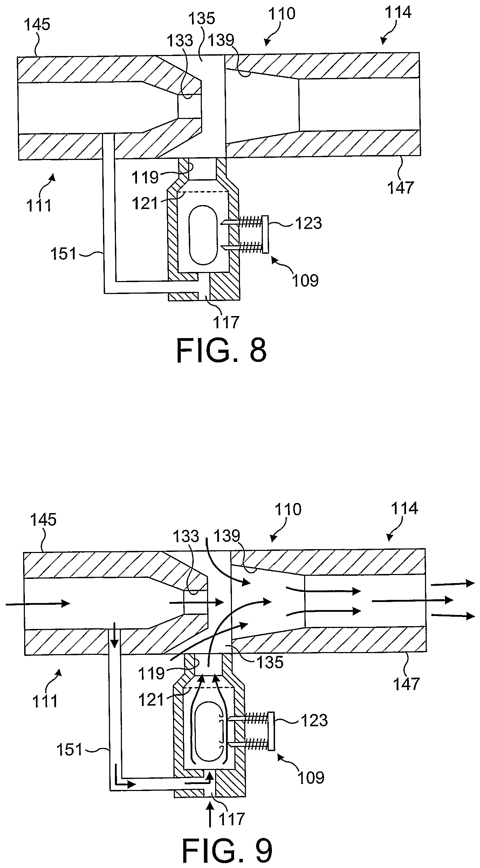

In one modification of the above-described delivery device, as illustrated in Figures 8 and 9, the substance supply unit 103 can be additionally fluidly connected to the mouthpiece unit 111, in this design by a flow channel 151 which fluidly connects the mouthpiece 145 to the inlet 117 of the substance supply unit 103, such as to provide for a supplemental air flow to the chamber 105, which assists in entraining the powdered substance as contained by the capsule 107.

By regulating this supplementary air flow and blending the same with the ambient air as entrained through the inlet 117 of the substance supply unit 103, the resulting air flow still has a reduced absolute humidity (water vapour content) as compared with an exhaled air flow, where the ambient air is not saturated.

Operation of this device, which is illustrated in Figure 9, is the same as for the delivery device of Figures 6 and 7.

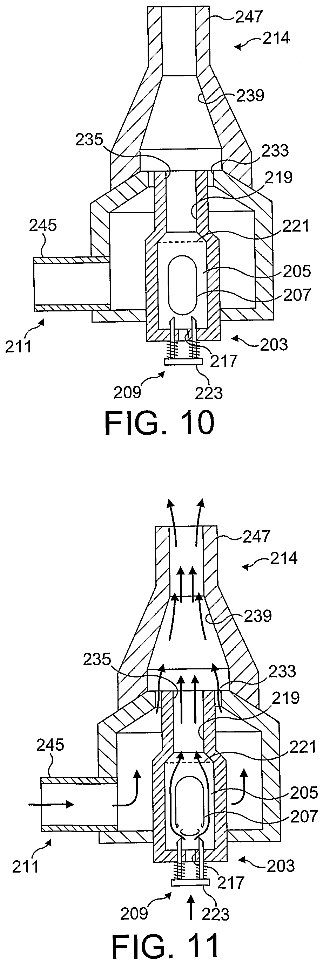

Figures 10 and 11 illustrate a yet further nasal delivery device.

The delivery device comprises a substance supply unit 203 which includes a chamber 205 which receives a capsule 207, which contains a metered amount of a powdered substance which is to be delivered by the delivery device, a rupturing mechanism 209 for rupturing the capsule 207, a Venturi unit 210 which is operative to draw an air flow of the ambient atmosphere through the chamber 205, a mouthpiece unit 211 which is in fluid communication with the Venturi unit 210 and is gripped in use in the mouth of a subject, and a nosepiece unit 214 which is in fluid communication with the Venturi unit 210 and is fitted to one nostril of the subject. For ease of illustration, the delivery device is illustrated in an orthogonal configuration, but, in its practical design, the mouthpiece unit 211 and the nosepiece unit 214 are configured for fitting to the mouth and one nostril of the subject.

The substance supply unit 203 includes an inlet 217 which fluidly connects the chamber 205 thereof with the ambient atmosphere and an outlet 219 which fluidly connects the chamber 205 thereof with the Venturi unit 210.

In this design the substance supply unit 203 includes a grid 221, here a gauze, which is disposed at the outlet 219 thereof and acts to prevent the capsule 207 or parts thereof from escaping from the chamber 205.

In this design the chamber 205 is cylindrical in shape.

In another design the chamber 205 could be spherical in shape, which is particularly advantageous in allowing for the release of the powdered substance from the capsule 207 when in any operative position.

In this design the chamber 205 and the grid 221, as components which contact the capsule 207 and the contained powdered substance, are fabricated from a material having a low moisture sensitivity, here a plastics material, such as to reduce any tendency to become tacky in the presence of moisture, and therefore reduce the tendency for the capsule 207 and the powdered substance as contained thereby to adhere to the wall of the chamber 205 or the grid 221.

In this design the rupturing mechanism 209 comprises a piercing element 223, here including two pins, which is operable to pierce the capsule 207, and thereby provide for the release of the contained powdered substance on the generation of a flow through the chamber 205.

In one design the capsule 207 is a gelatine capsule.

In another design the capsule 207 can be manufactured from a material which has a reduced tendency to become tacky in the presence of moisture, as occurs with gelatine capsules, and therefore reduce the tendency for the capsule 207 to adhere to the wall of the chamber 205 or the grid 221.

In one design the capsule 207 is formed of a cellulose derivative, such as hydroxypropyl methylcellulose (HPMC), hydroxypropylcellulose, methylcellulose, ethylcellulose and carboxymethylcellulose

In another design the capsule 207 can comprise a plastics material, preferably a water insoluble material, such as a polycarbonate.

In one design the capsule 207 can be manufactured from a lightweight material, such as thin-wall section polymeric materials, which reduces the energy required to move the capsule 207, typically by one or both of vibration and rotation, and thereby allows the delivery device to be operated at reduced flow rates, which is particularly advantageous for nasal delivery.

In one design the capsule 207 has a wall section of less than about 0.25 mm, and more preferably less than about 0.2 mm.

In an alternative design the capsule 207 can include an outer coating of a material which has a reduced tendency to become tacky in the presence of moisture, as occurs with gelatine capsules, and therefore reduce the tendency for the capsule 207 to adhere to the wall of the chamber 205 or the grid 221.

In one design the coated capsule 207 can be formed of gelatine.

In one design the coating can comprise one of parylene, hydroxypropyl methylcellulose (HPMC), hydroxypropylcellulose, methylcellulose, ethylcellulose, carboxymethylcellulose, polyvinyl alcohol, acrylic acid polymer, methacrylic acid polymer, ethyl acrylic acid polymer, cellulose acetate phthalate, polyvinyl acetate phthalate, hydroxypropyl methylcellulose phthalate and hydroxyl methylcellulose acetate succinate, or any combination of layers thereof.

The delivery device of this design is operative to discharge the powdered substance from the capsule 207 by rotation and vibration of the capsule 207, and thus the capsule 207 is preferably formed of a material or coated with a material which exhibits substantially no tackiness in the presence of a moist environment, here a saturated exhaled air flow, that is, does not exhibit an increased moisture content at the outer surface thereof, which would prevent reliable rotation and vibration of the capsule 207.

In this design the capsule 207 is cylindrical in shape, with hemispherical ends.

In other designs the capsule 207 could have other geometric forms, such as spherical, which allows for efficient powder release at low flow rates.

In one design the capsule 207 can comprise two or more parts.

In one alternative design the capsule 207 can be constructed to act as the primary environmental barrier for the powdered substance. For instance, the capsule 207 could be constructed from a relatively thick-walled cylindrical section of a polymeric material which includes two metalized thin film closure members which act to seal the ends of the cylindrical section and thus enclose the same.

The Venturi unit 210 comprises at least one driving air flow inlet 233 which is in fluid communication with the mouthpiece unit 211 and provides a constriction which acts to accelerate the exhaled air flow to deliver at least one driving air flow at a higher velocity, a second, substance air flow inlet 235 which is fluid communication with the outlet 219 of the substance supply unit 203 and through which, by the reduced local pressure as developed threat by the Venturi effect, is drawn a substance air flow from the chamber 205 of the substance supply unit 203 which entrains the powdered substance, and an air flow outlet 239 which is in fluid communication with the nosepiece unit 214 and through which the driving air flow and the substance air flow are delivered. In this design the at least one driving air flow is directed substantially parallel to the substance air flow.

In this design the Venturi unit 210 comprises a plurality of air flow inlets 233 which are disposed in an annular arrangement, here concentrically, about the substance air flow inlet 235.

This configuration, which utilizes ambient air to entrain the powdered substance from the capsule 207, is particularly advantageous, in avoiding the use of exhaled air to entrain the powdered substance. Exhaled air has a high humidity which would lead to condensation both in the chamber 205 and the capsule 207, which can cause problems in the complete entrainment of the powdered substance, both in terms of adhesion of the capsule 207 and the contained powdered substance to the wall of the chamber 205 and adhesion of the powdered substance to the capsule 207, particularly where the powdered substance is a hygroscopic powder.

The mouthpiece unit 211 comprises a mouthpiece 245, in this design as defined by a tubular section, which is gripped in the mouth of the subject.

The nosepiece unit 214 comprises a nosepiece 247, in this design as defined by a tubular section, which is inserted into a nostril of the subject, in this design to provide a sealing fit therewith.

In this design the nosepiece 247, as a component which contacts the powdered substance, is fabricated from a material having a low moisture sensitivity, here a plastics material, such as to reduce any tendency to become tacky in the presence of moisture, and therefore reduce the tendency for the powdered substance to adhere to the wall of the nosepiece 247.

In one design, where the delivery device is a reusable device, the chamber 205, which contains the capsule 207, and the nosepiece 247 comprise a unitary, replaceable component.

In operation, as illustrated in Figure 11, a subject operates the rupturing mechanism 209 to rupture the capsule 207, inserts the nosepiece 247 into one of his/her nostrils, grips the mouthpiece 245 in his/her mouth, and exhales through the mouthpiece 245.

The exhaled air flow is forced through the at least one driving air flow inlet 233 of the Venturi unit 210, which acts to deliver the exhaled air flow as a driving air flow past the substance air flow inlet 235 of the Venturi unit 210 and draw a substance air flow, which entrains powdered substance, from the chamber 205 of the substance supply unit 203. The substance air flow acts to move the capsule 207, in this design by vibration and rotation, and entrain the powdered substance as contained by the capsule 207.

The exhaled air flow, as then entraining the powdered substance, passes through the air flow outlet 239 of the Venturi unit 210, and is delivered though the nosepiece 247 into one nasal cavity of the subject.

In this design the exhaled air flow has such a pressure as to pass around the posterior margin of the nasal septum, and into the other nasal cavity, thereby achieving a bi-directional air flow as described in the applicants' earlier WO-A-00/051672.

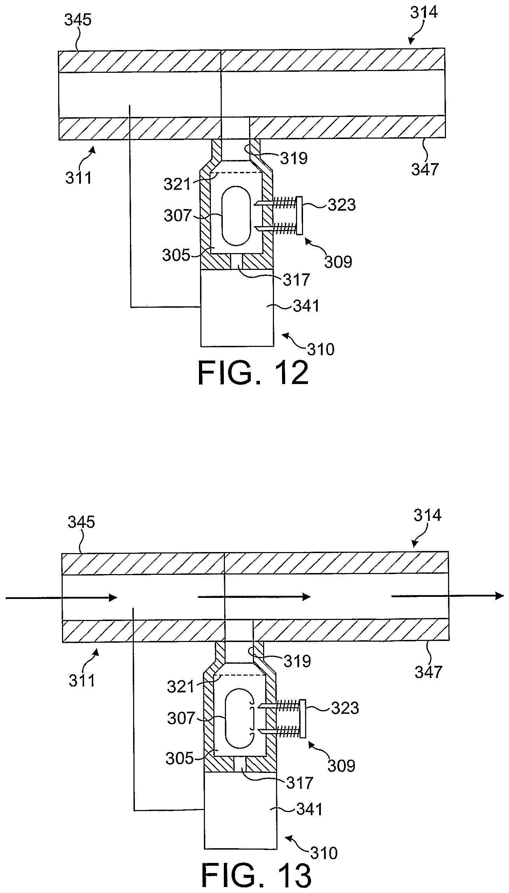

Figures 12 to 14 illustrate a still further nasal delivery device.

The delivery device comprises a substance supply unit 303 which includes a chamber 305 which receives a capsule 307, which contains a metered amount of a powdered substance which is to be delivered by the delivery device, a rupturing mechanism 309 for rupturing the capsule 307, a gas supply unit 310 which is operative to deliver a gas flow through the chamber 305, a mouthpiece unit 311 which is in fluid communication with the chamber 305 and is gripped in use in the mouth of a subject, and a nosepiece unit 314 which is in fluid communication with the chamber 305 and is fitted to one nostril of the subject. For ease of illustration, the delivery device is illustrated in an elongate configuration, but, in its practical design, the mouthpiece unit 311 and the nosepiece unit 314 are configured for fitting to the mouth and one nostril of the subject.

The substance supply unit 303 includes an inlet 317 which fluidly connects the chamber 305 thereof with the gas supply unit 310 and an outlet 319 which fluidly connects the chamber 305 thereof with the mouthpiece unit 311 and the nosepiece unit 314.

In this design the substance supply unit 303 includes a grid 321, here a gauze, which is disposed at the outlet 319 thereof and acts to prevent the capsule 307 or parts thereof from escaping from the chamber 305.

In this design the chamber 305 is cylindrical in shape.

In another design the chamber 305 could be spherical in shape, which is particularly advantageous in allowing for the release of the powdered substance from the capsule 307 when in any operative position.

In this design the chamber 305 and the grid 321, as components which contact the capsule 307 and the contained powdered substance, are fabricated from a material having a low moisture sensitivity, here a plastics material, such as to reduce any tendency to become tacky in the presence of moisture, and therefore reduce the tendency for the capsule 307 and the powdered substance as contained thereby to adhere to the wall of the chamber 305 or the grid 321.

In this design the rupturing mechanism 309 comprises a piercing element 323, here including two pins, which is operable to pierce the capsule 307, and thereby provide for the release of the contained powdered substance on the generation of a flow through the chamber 305.

In one design the capsule 307 is a gelatine capsule.

In another design the capsule 307 can be manufactured from a material which has a reduced tendency to become tacky in the presence of moisture, as occurs with gelatine capsules, and therefore reduce the tendency for the capsule 307 to adhere to the wall of the chamber 305 or the grid 321.

In one design the capsule 307 is formed of a cellulose derivative, such as hydroxypropyl methylcellulose (HPMC), hydroxypropylcellulose, methylcellulose, ethylcellulose and carboxymethylcellulose

In another design the capsule 307 can comprise a plastics material, preferably a water insoluble material, such as a polycarbonate.

In one design the capsule 307 can be manufactured from a lightweight material, such as thin-wall section polymeric materials, which reduces the energy required to move the capsule 307, typically by one or both of vibration and rotation, and thereby allows the delivery device to be operated at reduced flow rates, which is particularly advantageous for nasal delivery.

In one design the capsule 307 has a wall section of less than about 0.25 mm, and more preferably less than about 0.2 mm.

In an alternative design the capsule 307 can include an outer coating of a material which has a reduced tendency to become tacky in the presence of moisture, as occurs with gelatine capsules, and therefore reduce the tendency for the capsule 307 to adhere to the wall of the chamber 305 or the grid 321.

In one design the coated capsule 307 can be formed of gelatine.

In one design the coating can comprise one of parylene, hydroxypropyl methylcellulose (HPMC), hydroxypropylcellulose, methylcellulose, ethylcellulose, carboxymethylcellulose, polyvinyl alcohol, acrylic acid polymer, methacrylic acid polymer, ethyl acrylic acid polymer, cellulose acetate phthalate, polyvinyl acetate phthalate, hydroxypropyl methylcellulose phthalate and hydroxyl methylcellulose acetate succinate, or any combination of layers thereof.

The delivery device of this design is operative to discharge the powdered substance from the capsule 307 by rotation and vibration of the capsule 307, and thus the capsule 307 is preferably formed of a material or coated with a material which exhibits substantially no tackiness in the presence of a moist environment, here a saturated exhaled air flow, that is, does not exhibit an increased moisture content at the outer surface thereof, which would prevent reliable rotation and vibration of the capsule 307.

In this design the capsule 307 is cylindrical in shape, with hemispherical ends.

In other designs the capsule 307 could have other geometric forms, such as spherical, which allows for efficient powder release at low flow rates.

In one design the capsule 307 can comprise two or more parts.

In one alternative design the capsule 307 can be constructed to act as the primary environmental barrier for the powdered substance. For instance, the capsule 307 could be constructed from a relatively thick-walled cylindrical section of a polymeric material which includes two metalized thin film closure members which act to seal the ends of the cylindrical section and thus enclose the same.

In this design the gas supply unit 310 comprises a high-pressure reservoir 341, preferably at a pressure of from about 1 bar to about 10 bar, and more preferably at a pressure from about 2 bar to about 10 bar, which, when actuated, delivers a gas flow which acts to drive powder release from the capsule 307. In one design the reservoir 341 can be a pre-filled volume of gas at high-pressure, such as a pressurized canister which contains a propellant. In an alternative design the reservoir 341 can be charged using a pump mechanism.

In this design the gas supply unit 310 is configured such as to be actuated on the generation of a predetermined flow rate through the mouthpiece unit 311, typically a flow rate of from about 10 l/min to about 50 l/min.

In another design the gas supply unit 310 can be configured such as to be actuated on the generation of a predetermined pressure at the mouthpiece unit 311.

In a further design the gas supply unit 310 can be configured such as to be manually actuated.

This configuration is particularly advantageous, in avoiding the use of exhaled air to entrain the powdered substance, and in one design allowing the use of a dry gas. Exhaled air has a high humidity which would lead to condensation both in the chamber 305 and the capsule 307, which can cause problems in the complete entrainment of the powdered substance, both in terms of adhesion of the capsule 307 and the contained powdered substance to the wall of the chamber 305 and adhesion of the powdered substance to the capsule 307, particularly where the powdered substance is a hygroscopic powder.

The mouthpiece unit 311 comprises a mouthpiece 345, in this design as defined by a tubular section, which is gripped in the mouth of the subject.

The nosepiece unit 314 comprises a nosepiece 347, in this design as defined by a tubular section, which is inserted into a nostril of the subject, in this design to provide a sealing fit therewith.

In this design the nosepiece 347, as a component which contacts the powdered substance, is fabricated from a material having a low moisture sensitivity, here a plastics material, such as to reduce any tendency to become tacky in the presence of moisture, and therefore reduce the tendency for the powdered substance to adhere to the wall of the nosepiece 347.

In one design, where the delivery device is a re-usable device, the chamber 305, which contains the capsule 307, and the nosepiece 347 comprise a unitary, replaceable component.

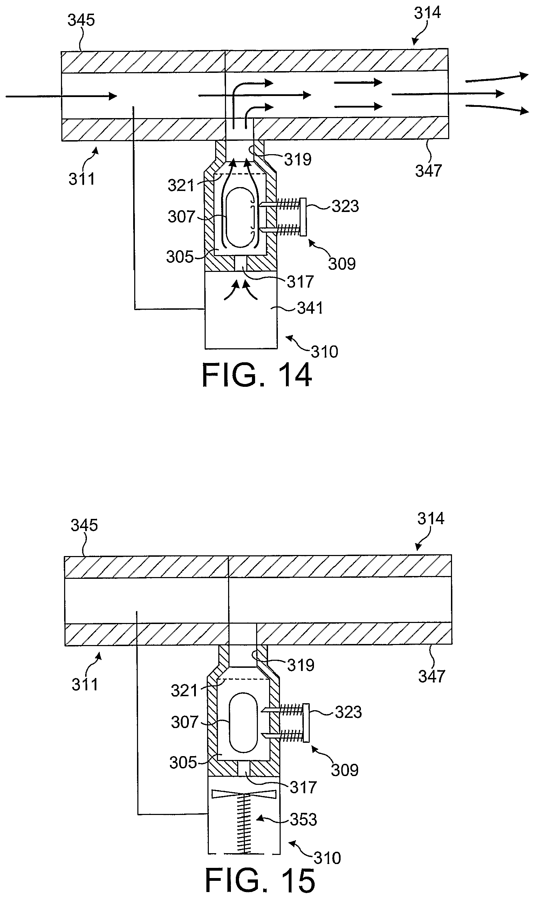

Operation of the delivery device will now be described hereinbelow with reference to Figures 13 and 14 of the accompanying drawings.

As illustrated in Figure 13, a subject operates the rupturing mechanism 309 to rupture the capsule 307, inserts the nosepiece 347 into one of his/her nostrils, grips the mouthpiece 345 in his/her mouth, and exhales through the mouthpiece 345.

The exhaled air flow is delivered though the nosepiece 347 into one nasal cavity of the subject.

In this design, as illustrated in Figure 14, when the exhaled air flow has a predetermined flow rate, the gas supply unit 310 is actuated, such as to deliver a gas flow through the chamber 305. This gas flow acts to move the capsule 307, in this design by vibration and rotation, and entrain the powdered substance as contained by the capsule 307, and the gas flow, as then entraining the powdered substance, is delivered into the exhaled air flow passing through the nosepiece 347 into one nasal cavity of the subject, such that the exhaled air flow entrains the powdered substance into the nasal cavity of the subject. This configuration is particularly advantageous where the gas supply unit 310 is a pressurized canister, as the gas flow from a pressurized canister is cold, and this cold gas is mixed with the warmer exhaled air flow prior to delivery to the nasal cavity.

In this design the exhaled air flow has such a pressure as to pass around the posterior margin of the nasal septum, and into the other nasal cavity, thereby achieving a bi-directional air flow as described in the applicants' earlier WO-A-00/051672.

In one modification, as illustrated in Figure 15, the gas supply unit 310 could comprise a charged turbine 353, for example, a propeller which is charged by a resilient element, such as spring. With this configuration, on actuation of the gas supply unit 310, stored energy drives the turbine to entrain atmospheric air through the chamber 305 which contains the capsule 307.

Finally, it will be understood that the present invention has been described in its preferred embodiments and can be modified in many different ways without departing from the scope of the invention as defined by the appended claims.

In one embodiment the powdered substance can also be formulated, for example, by coating or blending, such as to reduce the hygroscopicity and transiently increase the dissolution time, and thus reduce any loss of powdered substance in the device due to interaction with condensation on the internal surfaces of the device.

Also, the delivery devices of the described embodiments have been described in relation to the use of capsules 7. It is to be understood that the present invention has application with any kind of powder delivery system, including blisters and metering from bulk, and can be configured as a single-use or multi-use device.

Furthermore, the delivery device of the first-described embodiment could be modified to incorporate a thermoelectric device as the heat exchanger 27, for example, a device which utilizes the Peltier effect.

## Claims

1. A nasal delivery device for delivering substance to a nasal cavity of a subject, the delivery device comprising:

   a substance supply unit (3) for supplying a dose of substance to be delivered to the nasal cavity of the subject, the substance supply unit (3) including an inlet (17) and an outlet (19);
   a nosepiece unit (15) including a nosepiece (30) for fitting to a nasal cavity of the subject and being in fluid communication with the outlet (19) of the substance supply unit (3); and
   a mouthpiece unit (11) including a mouthpiece (25) in fluid communication with the inlet (17) of the substance supply unit (3) through which the subject in use exhales such as to entrain substance from the substance supply unit (3) and deliver the same through the nosepiece (30);
   at least one temperature modifier (27) for reducing a temperature of the exhaled air flow such as to reduce the absolute humidity thereof;
   **characterized by**:

   the substance supply unit (3) comprising a container chamber (5) for receiving a substance-containing container (7) which contains a dose of substance;
   the container chamber (5) and the nosepiece (30) comprising a unitary, replaceable component;
   the at least one temperature modifier (27) comprising a plurality of elongate channels (29);
   the plurality of elongate channels (29) allowing a flow therethrough at a flow rate of at least about 10 l/min at a pressure of less than about 2 kPa; and
   the plurality of elongate channels (29) providing a pressure drop of not more than about 0.5 kPa to the exhaled air flow.

2. The delivery device of claim 1, wherein the mouthpiece unit (11) includes a plurality of temperature modifiers (27a, b) which can be fluidly connected successively to the mouthpiece (25), and a switching mechanism (31) which allows for one of the temperature modifiers (27a, b) to be fluidly connected to the mouthpiece (25).

3. The delivery device of claim 2, wherein, when the one of the temperature modifiers (27a, b) is fluidly connected to the mouthpiece (25), the at least one other temperature modifier (27a, b) is vented to atmosphere.

4. The delivery device of claim 2 or 3, wherein the switching mechanism (31) comprises a rotatable member to which the temperature modifiers (27a, b) are disposed, whereby rotation of the switching mechanism (31) provides for the one of the temperature modifiers (27a, b) to be in fluid communication with the mouthpiece (25).

5. The delivery device of any of claims 1 to 4, wherein the plurality of elongate channels (29) allow a flow therethrough at a flow rate of at least about 10 l/min at a pressure of less than about 1 kPa.

6. The delivery device of any of claims 1 to 5, wherein the plurality of elongate channels (29) allow a flow therethrough at a flow rate of at least about 20 l/min, optionally at least about 30 l/min, optionally at least about 40 l/min and optionally at least about 50 l/min at a pressure of less than about 2 kPa, optionally less than about 1 kPa.

7. The delivery device of any of claims 1 to 6, wherein the plurality of elongate channels (29) provide a pressure drop of not more than about 0.25 kPa, optionally not more than about 0.10 kPa, optionally not more than about 0.05 kPa and optionally not more than about 0.025 kPa to the exhaled air flow.

8. The delivery device of any of claims 1 to 7, wherein the container chamber (5) is substantially cylindrical in shape.

9. The delivery device of any of claims 1 to 7, wherein the container chamber (5) is substantially spherical in shape.

10. The delivery device of any of claims 1 to 9, wherein the substance supply unit comprises a rupturing mechanism (9) for rupturing the container (7) as contained in the container chamber (5).

11. The delivery device of any of claims 1 to 10 in combination with a container (7), wherein the container (7) is formed substantially of a cellulose derivative.

12. The delivery device of claim 11, wherein the container (7) is formed substantially of one of hydroxypropyl methylcellulose (HPMC), hydroxypropylcellulose, methylcellulose, ethylcellulose and carboxymethylcellulose.

13. The delivery device of any of claims 1 to 10 in combination with a container (7), wherein the container (7) is formed substantially of gelatine.

14. The delivery device of any of claims 1 to 10 in combination with a container (7), wherein the container (7) is formed of a plastics material.

15. The delivery device of any of claims 1 to 10 in combination with a container (7), wherein the container (7) includes a coating comprising substantially one of parylene, hydroxypropyl methylcellulose (HPMC), hydroxypropylcellulose, methylcellulose, ethylcellulose, carboxymethylcellulose, polyvinyl alcohol, acrylic acid polymer, methacrylic acid polymer, ethyl acrylic acid polymer, cellulose acetate phthalate, polyvinyl acetate phthalate, hydroxypropyl methylcellulose phthalate and hydroxyl methylcellulose acetate succinate, or any combination of layers thereof.

16. The delivery device of claim 15, wherein the container (7) comprises a body of gelatine.

17. The delivery device of any of claims 1 to 16 in combination with a container (7), wherein the container (7) comprises a capsule.

18. The delivery device of claim 17, wherein the capsule is substantially cylindrical in shape.

19. The delivery device of claim 17, wherein the capsule is substantially spherical in shape.

**Patentansprüche**

1. Nasales Verabreichungsgerät zum Verabreichen einer Substanz an eine nasale Höhle eines Subjekts, wobei das Verabreichungsgerät umfasst:

eine Substanzliefereinheit (3) zum Liefern einer Dosis einer Substanz, die der nasalen Höhle des Subjekts verabreicht werden soll, wobei die Substanzliefereinheit (3) einen Einlass (17) und einen Auslass (19) aufweist;
eine Nasenstückeinheit (15) einschließlich eines Nasenstücks (30), das zu einer nasalen Höhle des Subjekts passt und in fluider Verbindung mit dem Auslass (19) der Substanzliefereinheit (3) steht; und
eine Mundstückeinheit (11) einschließlich eines Mundstücks (25), das in fluider Verbindung mit dem Einlass (17) der Substanzliefereinheit (3) steht und durch die das Subjekt bei der Verwendung ausatmet, um so Substanz aus der Substanzliefereinheit (3) mitzureißen und diese durch das Nasenstück (30) zu liefern;
wenigstens einen Temperaturveränderer (27) zum Verringern einer Temperatur des ausgeatmeten Luftflusses, um so dessen absolute Feuchtigkeit zu verringern,;

**gekennzeichnet durch**:

die Substanzliefereinheit (3) umfasst eine Containerkammer (5) zum Erhalten eines Substanz enthaltenden Containers (7), der eine Dosis der Substanz enthält;

die Containerkammer (5) und das Nasenstück (30) umfassen eine einheitliche, austauschbare Komponente;

der wenigstens eine Temperaturveränderer (27) umfasst mehrere Längskanäle (29);

die mehreren Längskanäle (29) ermöglichen durch sich einen Fluss in einer Flussrate von wenigstens 10 l/min bei einem Druck von weniger als 2 kPa; und

die mehreren Längskanäle (29) sorgen für den ausgeatmeten Luftfluss für einen Druckabfall von nicht mehr als 0,5 kPa.

2. Verabreichungsgerät nach Anspruch 1, wobei die Mundstückeinheit (11) mehrere Temperaturveränderer (27a, b) beinhaltet, die nacheinander mit dem Mundstück (25) in fluide Verbindung gebracht werden können, sowie einen Umschaltmechanismus (31), der es ermöglicht, dass einer der Temperaturveränderer (27a, b) mit dem Mundstück (25) in fluide Verbindung gebracht wird.

3. Verabreichungsgerät nach Anspruch 2, wobei, wenn der eine der Temperaturveränderer (27a, b) mit dem Mundstück (25) in fluide Verbindung gebracht wurde, der wenigstens eine andere Temperaturveränderer (27a, b) zur Atmosphäre entlüftet wird.

4. Verabreichungsgerät nach Anspruch 2 oder 3, wobei der Umschaltmechanismus (31) ein drehbares Element umfasst, an dem die Temperaturveränderer (27a, b) angeordnet sind, wobei eine Drehung des Umschaltmechanismus (31) dazu führt, dass einer der Temperaturveränderer (27a, b) mit dem Mundstück (25) in fluider Verbindung steht.

5. Verabreichungsgerät nach einem der Ansprüche 1-4, wobei die mehreren Längskanäle (29) durch sich einen Fluss in einer Flussrate von wenigstens 10 l/min bei einem Druck von weniger als 1 kPa ermöglichen.

6. Verabreichungsgerät nach einem der Ansprüche 1-5, wobei die mehreren Längskanäle (29) durch sich einen Fluss in einer Flussrate von wenigstens 20 l/min, wahlweise von wenigstens 30 l/min, wahlweise von wenigstens 40 l/min, und wahlweise von wenigstens 50 l/min bei einem Druck von weniger als 2 kPa, wahlweise weniger als 1 kPa ermöglichen.

7. Verabreichungsgerät nach einem der Ansprüche 1-6, wobei die mehreren Längskanäle (29) für den ausgeatmeten Luftfluss für einen Druckabfall von nicht mehr als ungefähr 0,25 kPa sorgen, wahlweise nicht mehr als ungefähr 0,1 kPa, wahlweise nicht mehr als ungefähr 0,05 kPa, und wahlweise nicht mehr als ungefähr 0,025 kPa.

8. Verabreichungsgerät nach einem der Ansprüche 1-7, wobei die Containerkammer (5) eine im Wesentlichen zylindrische Form aufweist.

9. Verabreichungsgerät nach einem der Ansprüche 1-7, wobei die Containerkammer (5) eine im Wesentlichen kugelförmige Form aufweist.

10. Verabreichungsgerät nach einem der Ansprüche 1-9, wobei die Substanzliefereinheit einen Aufreißmechanismus (9) zum Aufreißen des in der Containerkammer (5) enthaltenen Containers (7) umfasst.

11. Verabreichungsgerät nach einem der Ansprüche 1-10 in Verbindung mit einem Container (7), wobei der Container (7) im Wesentlichen aus einem Zellulosederivat gebildet ist.

12. Verabreichungsgerät nach Anspruch 11, wobei der Container (7) im Wesentlichen aus einem von Hydroxypropyl-Methylzellulose (HPMC), Hydroxypropylzellulose, Methylzellulose, Ethylzellulose und Carboxymethylzellulose gebildet ist.

13. Verabreichungsgerät nach einem der Ansprüche 1-10 in Verbindung mit einem Container (7), wobei der Container (7) im Wesentlichen aus Gelatine gebildet ist.

14. Verabreichungsgerät nach einem der Ansprüche 1-10 in Verbindung mit einem Container (7), wobei der Container (7) im Wesentlichen aus einem Kunststoffmaterial gebildet ist.

15. Verabreichungsgerät nach einem der Ansprüche 1-10 in Verbindung mit einem Container (7), wobei der Container (7) eine Beschichtung beinhaltet, umfassend im Wesentlichen eines von Parylen, Hydroxypropyl-Methylzellulose (HPMC), Hydroxypropylzellulose, Methylzellulose, Ethylzellulose, Carboxymethylzellulose, Polyvinylalkohol, Akrylsäurepolymer, Methakrylsäurepolymer, Ethylakrylsäurepolymer, Zelluloseacetatphtalat, Polyvinylacetatphtalat, Hydroxypropyl-Methylzellulosephtalat, und Hydroxypropyl-Methylzelluloseacetatsuccinat oder jegliche Kombination davon.

16. Verabreichungsgerät nach Anspruch 15, wobei der Container (7) einen Körper aus Gelatine umfasst.

**17.** Verabreichungsgerät nach einem der Ansprüche 1-16 in Verbindung mit einem Container (7), wobei der Container (7) eine Kapsel umfasst.

**18.** Verabreichungsgerät nach Anspruch 17, wobei die Kapsel eine im Wesentlichen zylindrische Form aufweist.

**19.** Verabreichungsgerät nach Anspruch 17, wobei die Kapsel eine im Wesentlichen kugelförmige Form aufweist.

**Revendications**

**1.** Dispositif d'administration nasale pour administrer une substance dans une cavité nasale d'un sujet, le dispositif d'administration comprenant :

une unité de distribution de substance (3) pour fournir une dose de substance à administrer dans la cavité nasale du sujet, l'unité de distribution de substance (3) comprenant une entrée (17) et une sortie (19) ;

une unité d'embout de nez (15) comprenant un embout de nez (30) pour une adaptation à une cavité nasale du sujet et une communication fluidique avec la sortie (19) de l'unité de distribution de substance (3) ; et

une unité d'embout buccal (11) comprenant un embout buccal (25) en communication fluidique avec l'entrée (17) de l'unité de distribution de substance (3), à travers laquelle le sujet, en utilisation, expire, de façon à entraîner une substance à partir de l'unité de distribution de substance (3) et administrer celle-ci à travers l'embout de nez (30) ;

au moins un modificateur de température (27) pour réduire une température du flux d'air expiré de façon à réduire l'humidité absolue de celui-ci ;

**caractérisé par** :

l'unité de distribution de substance (3) comprenant une chambre de récipient (5) pour recevoir un récipient de réception de substance (7) qui contient une dose de substance ;

la chambre de récipient (5) et l'embout de nez (30) comprenant un composant unitaire remplaçable ;

l'au moins un modificateur de température (27) comprenant une pluralité de canaux allongés (29) ;

la pluralité de canaux allongés (29) permettant un flux à travers ceux-ci à un débit d'au moins environ 10 l/min à une pression de moins d'environ 2 kPa ; et

la pluralité de canaux allongés (29) fournissant une chute de pression de pas plus d'environ 0,5 kPa au flux d'air expiré.

**2.** Dispositif d'administration selon la revendication 1, dans lequel l'unité d'embout buccal (11) comprend une pluralité de modificateurs de température (27a,b) qui peuvent être reliés fluidiquement de manière successive à l'embout buccal (25), et un mécanisme de commutation (31) qui permet à l'un des modificateurs de température (27a,b) d'être relié fluidiquement à l'embout buccal (25).

**3.** Dispositif d'administration selon la revendication 2, dans lequel, lorsque celui des modificateurs de température (27a,b) est relié fluidiquement à l'embout buccal (25), l'au moins un autre modificateur de température (27a,b) est mis à l'air libre.

**4.** Dispositif d'administration selon la revendication 2 ou 3, dans lequel le mécanisme de commutation (31) comprend un élément rotatif sur lequel les modificateurs de température (27a,b) sont disposés, ce par quoi une rotation du mécanisme de commutation (31) amène l'un des modificateurs de température (27a,b) à être en communication fluidique avec l'embout buccal (25).

**5.** Dispositif d'administration selon l'une quelconque des revendications 1 à 4, dans lequel la pluralité de canaux allongés (29) permettent un flux à travers ceux-ci à un débit d'au moins environ 10 1/min à une pression de moins d'environ 1 kPa.

**6.** Dispositif d'administration selon l'une quelconque des revendications 1 à 5, dans lequel la pluralité de canaux allongés (29) permettent un flux à travers ceux-ci à un débit d'au moins environ 20 l/min, facultativement d'au moins environ 30 l/min, facultativement d'au moins environ 40 l/min, et facultativement d'au moins environ 50 l/min, à une pression de moins d'environ 2 kPa, facultativement de moins d'environ 1 kPa.

**7.** Dispositif d'administration selon l'une quelconque des revendications 1 à 6, dans lequel la pluralité de canaux allongés (29) fournissent une chute de pression de pas plus d'environ 0,25 kPa, facultativement de pas plus d'environ 0,10 kPa, facultativement de pas plus d'environ 0,05 kPa, et facultativement de pas plus d'environ 0,025 kPa, au flux d'air expiré.

**8.** Dispositif d'administration selon l'une quelconque des revendications 1 à 7, dans lequel la chambre de récipient (5) est de forme sensiblement cylindrique.

**9.** Dispositif d'administration selon l'une quelconque des revendications 1 à 7, dans lequel la chambre de récipient (5) est de forme sensiblement sphérique.

**10.** Dispositif d'administration selon l'une quelconque des revendications 1 à 9, dans lequel l'unité de distribution de substance comprend un mécanisme de rupture (9) pour rompre le récipient (7) contenu dans la chambre de récipient (5).

**11.** Dispositif d'administration selon l'une quelconque des revendications 1 à 10 en combinaison avec un récipient (7), le récipient (7) étant sensiblement formé d'un dérivé de cellulose.

**12.** Dispositif d'administration selon la revendication 11, dans lequel le récipient (7) est sensiblement formé de l'une parmi l'hydroxypropylméthylcellulose (HPMC), l'hydroxypropylcellulose, la méthylcellulose, l'éthylcellulose et la carboxyméthylcellulose.

**13.** Dispositif d'administration selon l'une quelconque des revendications 1 à 10 en combinaison avec un récipient (7), le récipient (7) étant sensiblement formé de gélatine.

**14.** Dispositif d'administration selon l'une quelconque des revendications 1 à 10 en combinaison avec un récipient (7), le récipient (7) étant formé d'une matière plastique.

**15.** Dispositif d'administration selon l'une quelconque des revendications 1 à 10 en combinaison avec un récipient (7), le récipient (7) comprenant un revêtement comprenant sensiblement l'un parmi le parylène, l'hydroxypropylméthylcellulose (HPMC), l'hydroxypropylcellulose, la méthylcellulose, l'éthylcellulose, la carboxyméthylcellulose, le poly(alcool de vinyle), un polymère d'acide acrylique, un polymère d'acide méthacrylique, un polymère d'acide éthylacrylique, l'acétophtalate de cellulose, l'acétophtalate de polyvinyle, le phtalate d'hydroxypropylméthylcellulose et le succinate d'acétate d'hydroxylméthylcellulose, ou toute combinaison de couches de ceux-ci.

**16.** Dispositif d'administration selon la revendication 15, dans lequel le récipient (7) comprend un corps de gélatine.

**17.** Dispositif d'administration selon l'une quelconque des revendications 1 à 16 en combinaison avec un récipient (7), le récipient (7) comprenant une capsule.

**18.** Dispositif d'administration selon la revendication 17, dans lequel la capsule est de forme sensiblement cylindrique.

**19.** Dispositif d'administration selon la revendication 17, dans lequel la capsule est de forme sensiblement sphérique.

FIG. 1

WARM EXHALED AIR ⇒    ⇒ COOLED EXHALED AIR

FIG. 2

FIG. 3

FIG. 4

FIG. 5

FIG. 6

FIG. 7

FIG. 8

FIG. 9

247
214
239
235
233
219
245
221
205
207
203
211
209 217
223

FIG. 10

247
214
239
235
233
219
245
221
205
207
211
203
209 223
217

FIG. 11

345   314

311   319   321   307   305   317   341   323   309   310

FIG. 12

345   314

311   319   321   307   305   317   341   323   309   310

FIG. 13

FIG. 14

FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4013075 A
- US 4889114 A
- WO 00051672 A
- WO 2003090812 A
- WO 2004103447 A
- GB 2404867 A
- WO 2004060433 A
- US 5373841 A